# FASCICULE DE BREVET EUROPEEN

(11) **EP 0 623 325 B1**
(45) Date de publication et mention de la délivrance du brevet: **04.03.1998**
(21) Numéro de dépôt: 94400895.2
(22) Date de dépôt: 26.04.1994
(51) Int. Cl.: A61F 5/03

(54) **Ceinture de contention dorso-abdominale**
Rücken-Bauchbezüglicher Stützgürtel
Dorsoabdominal support belt

(30) Priorité: 04.05.1993 FR 9305286
(43) Date de publication de la demande: 09.11.1994
(73) Titulaire: Zagame, André, F-61110 Rémalard (FR)
(72) Inventeur: Zagame, André, F-61110 Rémalard (FR)
(74) Mandataire: Jaunez, Xavier

(56) Documents cités:
- FR-A- 487 766
- FR-A- 617 964
- FR-A- 1 213 302
- US-A- 1 661 720
- US-A- 2 584 279
- US-A- 2 586 658
- US-A- 2 765 470

## Description

La présente invention concerne les ceintures de contention dorso-abdominales, qui sont constituées par un ensemble unitaire à patte d'entre-jambes agencé pour exercer une compression permanente de la zone concernée du corps, conformément au préambule de la revendication 1.

Le terme "ceinture" doit être compris dans un sens large dans le cadre de l'invention, c'est-à-dire englobant les gaines basses, les gaines hautes, et les différents types d'ensembles se prolongeant vers le bas (jusqu'en dessous des genoux) et/ou vers le haut (avec éventuellement deux bretelles de maintien).

De telles ceintures sont couramment utilisées pour faire disparaître au moins en partie des marques ou cicatrices formant des bourrelets de tissu fibreux, qui subsistent après le traitement des lésions profondes de la peau. Elles sont en particulier recommandées par les spécialistes de la chirurgie obstétrique, digestive ou viscérale, plastique ou reconstructive.

Les ceintures de contention traditionnelles sont alors réalisées sous la forme d'un ensemble unitaire en matériau textile élastique spécialement conçu pour exercer une compression permanente, et aussi contrôlée que possible, sur la zone concernée du corps, et en particulier la zone dorso-abdominale. Certaines ceintures plus élaborées, spécialement choisies en post-opératoire, comportent une patte d'entre-jambes, munie d'une fermeture à crochets, ayant pour effet que la tension tend à s'exercer à la fois horizontalement et verticalement. On obtient ainsi un ensemble unitaire qui est à la fois efficace quant à son effet thérapeutique anti-oedémateux, et esthétique pour la personne qui porte cette ceinture de contention.

Cependant, dans certaines situations, ces ceintures deviennent inadaptées, voire impropres à un port prolongé. C'est notamment le cas des femmes enceintes, dans la mesure où le développement de l'abdomen vient perturber la répartition des tensions du matériau textile élastique.

En effet, si la ceinture dorso-abdominale a été choisie pour exercer une contention contrôlée en début de grossesse, le développement de l'abdomen a pour effet d'induire localement une pression de contention dépassant largement 25 mm Hg, avec un risque de blocage des fonctions musculaires, et avec une perte de contrôle de la contention exercée. Si par contre la ceinture a été choisie en tenant compte du développement futur de l'abdomen, alors la contention exercée en début de grossesse sera généralement insuffisante, et de plus le contrôle ultérieur de la contention exercée deviendra de plus en plus aléatoire au fur et à mesure de l'avancement de la grossesse. Dans ce dernier cas, on risque de rencontrer encore plus fréquemment des syndromes du type syndrome de Lacome (syndrome ostéo-ligamento-musculaire), avec apparition de fatigues et/ou de douleurs, et la personne éprouve un besoin impératif de soulagement que la ceinture ne peut pas lui apporter.

L'invention a précisément pour but de résoudre ce problème, en concevant une ceinture de contention dorso-abdominale ne présentant pas les inconvénients et limitations précités.

L'homme de l'art pourrait être tenté de s'inspirer des ceintures de confort qui existent déjà pour répondre au cas particulier des femmes enceintes. Ces ceintures sont cependant exclusivement conçues comme ceintures de confort : en effet, on utilise en général des laçages (simples ou doubles) agencés verticalement, pour faire varier le périmètre abdominal total, ce qui est totalement antinomique avec la recherche d'une action thérapeutique avec contention contrôlée (au contraire, les laçages rigides induisent un risque de blocage des fonctions musculaires du fait d'une compression excessive), sans parler naturellement du matériau utilisé, plus ou moins épais, non conçu pour exercer une compression permanente sur la zone concernée du corps.

De ce fait, les produits existants dans la lingerie spécialisée pour femmes enceintes ne sont d'aucune aide pour résoudre le problème précité en rapport avec l'exercice d'une contention contrôlée.

Pour compléter l'état de la technique, on peut encore citer quelques solutions très anciennes, dont le port serait d'ailleurs aujourd'hui interdit du fait de leur incapacité à contrôler la contention exercée, laquelle contention est d'ailleurs presque exclusivement circulaire, ce qui induit un risque de blocage des fonctions musculaires.

Le document US-A-2,586,658 décrit ainsi une ceinture dorsale constituée par une partie dorso-postérieure en matériau non-élastique et une partie antérieure en tissu élastique, avec des sangles portées en bretelles et venant s'accrocher sur un gousset pubien cousu sur cette partie antérieure. La contention exercée par une telle ceinture est seulement circulaire du fait de l'absence de patte d'entre-jambes, et surtout ne peut être contrôlée en raison du caractère non-élastique de la partie dorso-postérieure. De plus, les sangles de cette ceinture sont anti-anatomiques, car les fixations dorsales sont placées très haut par rapport aux vertèbres lombaires L4, L5. Enfin, une telle conception serait exclue pour une femme enceinte, car l'accrochage des sangles sur un gousset pubien constitue un sanglage rigide passant sur l'abdomen, de sorte qu'il y aurait un danger certain pour la femme enceinte.

Le document FR-E-20 728 décrit une ceinture de grossesse qui est une simple sangle abdominale de maintien à bandes élastiques servant à soulager l'abdomen au niveau pubien. Une telle sangle ne peut exercer une véritable action thérapeutique de contention. En outre, la présence de boucles de fixation en tissu non élastique rend impossible tout contrôle de la pression exercée par les bandes élastiques.

Le document FR-A-617.964 décrit un maillot de grossesse en tissu à côtes sur lequel sont fixées par des coutures des bandes plates antérieure et postérieure inextensibles et des sangles plates obliques en tissu élastique extra-fort. Un tel maillot permet d'exercer une contention qui est seulement circulaire, et qui risque de bloquer les grands muscles. Les bandes inextensibles sont en particulier contre-indiquées pour le libre jeu des fonctions musculaires (d'ailleurs, même en cas de déficience musculaire, le blocage des fonctions musculaires se traduit par une atrophie de ces fonctions).

L'arrière-plan technologique peut être enfin illustré en citant les documents US-A-2,765,470, FR-A-1.213.302, US-A-1,661,720 et US-A-2,584,279.

L'invention a ainsi pour objet de réalisaer une ceinture dorso-abdominale capable de tolérer un certain développement volumique du patient tout en gardant le contrôle de la contention exercée de façon uniforme et multidirectionnelle, et ce sans risque de compression excessive, même locale, pour la personne qui porte cette ceinture.

Ce problème est résolu conformément à l'invention grâce à une ceinture dorso-abdominale du type précité, dans laquelle l'ensemble unitaire se compose de deux parties complémentaires se raccordant par une couture associée, dont une partie principale antéro-postérieure enveloppante réalisée en un premier matériau textile élastique choisi pour exercer une contention contrôlée, et une partie abdominale réalisée en un second matériau textile élastique qui est sensiblement plus extensible que ledit premier matériau, de façon que cette partie abdominale permette un développement plus libre de l'abdomen, et dans laquelle un bandeau périphérique est relié à l'ensemble unitaire par des coutures concernant la zone centrale antérieure et/ou la zone centrale postérieure de ce bandeau, le restant dudit bandeau reposant librement contre la surface dudit ensemble unitaire, afin que ce bandeau périphérique exerce une contention supplémentaire de la zone sus-pubienne et/ou pré-ombilicale, conformément à la partie caractérisante de la revendication 1.

De préférence, la partie abdominale de l'ensemble unitaire se raccorde à la partie principale dudit ensemble par une couture en forme de U dont la partie incurvée est essentiellement recouverte par la partie frontale du bandeau périphérique.

Avantageusement en outre, l'ensemble unitaire comporte sur un côté une ligne d'ouverture à crochets visant à faciliter la mise en place de ladite ceinture. En particulier, la ligne d'ouverture à crochets comporte plusieurs rangées de façon à pouvoir régler le dimensionnement périmétrique de ladite ceinture.

Il peut s'avérer également intéressant que l'ensemble unitaire soit renforcé par un baleinage en vue d'un meilleur soutien, avec de préférence deux baleines antérieures et deux baleines postérieures.

Avantageusement encore, les coutures reliant le bandeau périphérique à l'ensemble unitaire forment au moins une ligne de fixation centrale antérieure et au moins une ligne de fixation centrale postérieure, ces deux lignes étant les seuls points de liaison entre ledit bandeau et ledit ensemble unitaire. En particulier, la ligne de fixation centrale antérieure est agencée dans la zone suspubienne de l'ensemble unitaire, et la ligne de fixation centrale postérieure est agencée dans la zone dorsale haute dudit ensemble unitaire.

Il est également intéressant que le bandeau périphérique présente, de chaque côté, une ligne d'ouverture à crochets à plusieurs rangées, de façon à pouvoir régler le dimensionnement périmétrique dudit bandeau.

Avantageusement enfin, le bandeau périphérique est réalisé en matériau textile élastique, ce matériau étant de préférence essentiellement identique au premier matériau qui constitue l'ensemble unitaire.

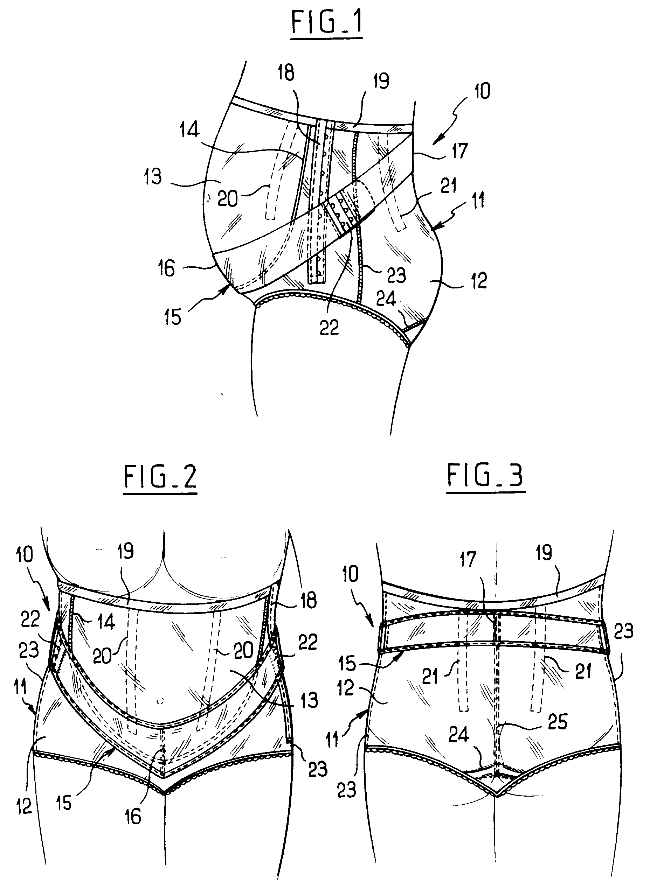

D'autres caractéristiques et avantages de l'invention apparaîtront plus clairement à la lumière de la description qui va suivre et du dessin annexé, en référence aux figures 1 à 3 illustrant, respectivement de profil, de face et de dos, une ceinture de contention dorso-abdominale conforme à l'invention, en l'espèce du type gaine basse, portée par une femme enceinte.

Les figures 1 à 3 illustrent une ceinture de contention dorso-abdominale 10 conforme à l'invention, qui est constituée par un ensemble unitaire 11 agencé pour exercer une compression permanente de la zone concernée du corps, ledit ensemble comportant une patte d'entre-jambes (non visible sur les figures) qui est de préférence équipée d'une fermeture à crochets, de préférence réglable, ayant pour effet que la tension tend à s'exercer à la fois horizontalement et verticalement.

Conformément à une caractéristique de l'invention, l'ensemble unitaire 11 se compose de deux parties complémentaires 12, 13 se raccordant par une couture associée 14, dont une partie principale antéro-postérieure enveloppante 12 et une partie abdominale 13, ces deux parties étant réalisées en des matériaux textiles dont les caractéristiques d'élasticité diffèrent. En effet, la partie principale antéro-postérieure enveloppante 12 est réalisée en un premier matériau textile élastique choisi pour exercer une contention bi-directionnelle contrôlée, tandis que la partie abdominale 13 est réalisée en un second matériau textile élastique qui est notablement plus extensible que le premier matériau, de façon que cette partie abdominale 13 permette un développement plus libre de l'abdomen. Ainsi que cela est mieux visible sur la figure 2, la partie abdominale 13 de l'ensemble unitaire 11 se raccorde à la partie principale 12 dudit ensemble par un contour 14, c'est-à-dire une couture de liaison, en forme de U, dont la partie incurvée est essentiellement recouverte par la partie frontale d'un bandeau périphérique 15 associé à l'ensemble unitaire 11, lequel bandeau sera décrit plus loin plus en détail tant pour sa structure que pour sa fonction. On utilisera de préférence comme matériau constitutif de la partie principale antéro-postérieure enveloppante 12 un matériau textile élastique du type lycra traditionnellement utilisé pour les ceintures de contention existantes, notamment pour le traitement des hypertrophies cicatricielles des grands brûlés (cas où la contention uniforme contrôlée est essentielle). Un tel matériau permet d'exercer une pression contrôlée en général comprise entre 16 et 24 mm Hg. Par contre, la partie abdominale 13 sera réalisée en un matériau plus souple, par exemple un lycra sensiblement plus extensible exerçant une contention ne dépassant pas 15 mm Hg.

Une telle structure en deux parties complémentaires permet à la fois de contrôler la contention exercée, et autorise un développement progressif de l'abdomen, sans générer, pour la zone abdominale, une compression excessive pour la personne qui porte la ceinture de contention dorso-abdominale. Il devient alors possible de choisir pour la personne concernée la ceinture de contention ayant la bonne taille, en début ou en cours de grossesse, sans que le développement abdominal ultérieur ne risque d'entraîner une compression excessive grâce à la grande extensibilité de la partie abdominale de l'ensemble unitaire.

En général, on prévoira cinq ou six tailles différentes pour avoir une adaptation parfaite aux différentes tailles de bassins. La contention contrôlée exigera dans la pratique un minimum de cinq ou six tailles différentes, afin de pouvoir adapter la ceinture, avec une efficacité optimale, aux différentes tailles et morphologies de bassins. Dans certains cas extrêmes où la morphologie de la patiente ne serait pas standard, il serait alors indispensable de réaliser la ceinture aux mesures de la patiente.

Conformément à une autre caractéristique de l'invention, un bandeau périphérique 15 est associé à l'ensemble unitaire 11, en étant relié audit ensemble par des coutures qui sont agencées de telle façon que ce bandeau périphérique puisse exercer une contention supplémentaire de la zone sus-pubienne et/ou pré-ombilicale. Ainsi que cela est mieux visible sur la figure 1, le bandeau périphérique 15 est agencé à la façon d'une sous-ventrière combinant à la fois l'effet de soutien abdominal et l'action thérapeutique avec contention contrôlée

L'ensemble unitaire 11 est ici réalisé sous la forme d'une gaine courte, en se terminant supérieurement par une bande élastique 19, mais ceci ne constitue naturellement qu'un exemple . Les différentes pièces constitutives de la partie principale antéro-postérieure enveloppante 12 sont liées entre elles par deux coutures latérales 23, et une couture postérieure 25 (visible seulement sur la figure 3), avec éventuellement des coutures annexes plus courtes telles que la couture inférieure 24 se raccordant à la couture postérieure précitée 25. Cependant, plus importante que les coutures 23, 24, 25 précitées est la couture 14 précitée, en forme de U, qui raccorde la partie principale antéro-postérieure enveloppante 12 et la partie abdominale 13.

Ainsi que cela est mieux visible sur la figure 1, il est avantageux de prévoir que l'ensemble unitaire 11 comporte, sur un côté, une ligne d'ouverture à crochets 18 visant à faciliter la mise en place de la ceinture de contention. La ligne d'ouverture à crochets 18 est disposée latéralement, ce qui est favorable au regard du blocage des fonctions musculaires, en formant une ouverture dans la partie antéro-postérieure enveloppante 12, selon une direction essentiellement verticale. De préférence, il sera prévu deux ou trois rangées verticales de crochets, de façon à prévoir un réglage périmétrique plus fin.

Par ailleurs, ainsi que cela est classique dans le domaine des ceintures de contention dorso-abdominales, il pourra s'avérer intéressant de prévoir un renforcement de l'ensemble unitaire 11 par un baleinage en vue d'un meilleur soutien, avec de préférence deux baleines antérieures 20 et deux baleines postérieures 21, ces baleines étant noyées dans une couture intérieure associée, en étant recouvertes par une épaisseur interne de tissu mou pour un meilleur confort.

Ainsi que cela a été dit plus haut, et conformément à une autre caractéristique de l'invention, un bandeau périphérique 15 est associé à l'ensemble unitaire 11, en étant relié audit ensemble par des coutures qui sont agencées de telle façon que ce bandeau puisse exercer une contention supplémentaire de la zone sus-pubienne et/ou pré-ombilicale.

On pourrait prévoir une liaison par une couture unique, mais ceci risquerait de rendre possibles des déplacements indésirables du bandeau en fonction des mouvements de la personne qui porte la ceinture de contention, et de nuire ainsi à l'action thérapeutique de contention que l'on souhaite exercer dans une zone précise subabdominale. C'est pour cela que l'on a prévu ici une liaison entre le bandeau périphérique 15 et l'ensemble unitaire 11 par des coutures 16, 17 concernant la zone centrale antérieure et/ou la zone centrale postérieure de ce bandeau, le restant dudit bandeau reposant librement contre la surface dudit ensemble unitaire. L'organisation de deux coutures 16, 17 apparaît ainsi préférable à l'utilisation d'une seule de ces deux coutures, qui concernerait alors soit la zone centrale antérieure, soit la zone centrale postérieure du bandeau périphérique. Il est apparu que l'on obtient une action thérapeutique optimale en prévoyant que les coutures 16, 17 forment au moins une ligne de fixation centrale antérieure et au moins une ligne de fixation centrale postérieure, ces deux lignes étant les seuls points de liaison entre le bandeau périphérique 15 et l'ensemble unitaire 11. Plus précisément, la ligne de fixation centrale antérieure 16 (visible sur les figures 1 et 2) est agencée dans la zone sus-pubienne de l'ensemble unitaire 11, tandis que la ligne de fixation centrale postérieure 17 (visible sur les figures 1 et 3) est agencée dans la zone dorsale haute dudit ensemble unitaire. Un tel agencement permet à la fois de maintenir l'orientation correcte du bandeau périphérique 15, orientation particulière du type sous-ventrière qui est bien visible sur la figure 1, tout en contrôlant parfaitement les forces exercées par ce bandeau légèrement étiré, en vue de la contention supplémentaire exercée par ledit bandeau.

Il va de soi que l'on pourra remplacer les coutures 16 et 17 par deux ou plusieurs coutures parallèles adjacentes, en s'attachant toutefois à préserver la disposition dans le plan médian de symétrie au niveau d'une part de la zone sus-pubienne de l'ensemble unitaire, et d'autre part de la zone dorsale haute dudit ensemble unitaire.

Il sera en général avantageux de prévoir une possibilité de réglage du dimensionnement périmétrique du bandeau périphérique. Une telle possibilité a été illustrée ici, et l'on constate, sur les figures 1 et 2, que le bandeau périphérique 15 présente, de chaque côté, une ligne d'ouverture à crochets 22 à plusieurs rangées, ce qui permet de régler le dimensionnement périmétrique dudit bandeau. On pourra prévoir ainsi une succession de cinq ou six rangées parallèles de crochets, ce qui permet d'effectuer un réglage fin de la partie droite et de la partie gauche du bandeau périphérique 15, indépendamment l'une de l'autre. Grâce à un positionnement prédéterminé des rangées choisi en fonction de l'augmentation de volume de l'abdomen pendant la grossesse, on dispose de réglages quantifiés permettant à la personne de relâcher très progressivement la tension exercée par le bandeau, par exemple en prenant chaque mois une nouvelle rangée de crochets à partir de la vingtième semaine de grossesse. On pourra naturellement remplacer les crochets par des moyens d'accrochage équivalents, par exemple des moyens auto-agrippants.

Pour la réalisation du bandeau périphérique 15, on utilisera de préférence un matériau textile élastique qui est essentiellement identique au matériau constituant l'ensemble unitaire 11, c'est-à-dire par exemple un lycra parfaitement adapté pour exercer une contention avec une pression comprise entre 17 et 21 mm Hg.

Ainsi que cela est aisé à comprendre, la ceinture de contention dorso-abdominale 10 peut être enfilée aisément, après avoir ouvert la ligne d'ouverture à crochets 18, et éventuellement aussi ouvert, ou à tout le moins desserré, au moins une ligne d'ouverture à crochets 22 du bandeau périphérique 15. Une fois la ceinture de contention mise en place à la bonne hauteur, il est alors aisé de refermer les lignes de fixation précitées de façon à obtenir l'action thérapeutique désirée avec une contention parfaitement contrôlée sur toute la zone du corps qui est enveloppée.

On est ainsi parvenu à réaliser une ceinture dorso-abdominale capable de tolérer un certain développement volumique du patient, en particulier un développement abdominal pour une femme enceinte, tout en gardant le contrôle de la contention exercée, et ce sans risque de compression excessive, même locale, pour la personne qui porte cette ceinture.

La ceinture de contention selon l'invention est ainsi applicable dans le cadre de nombreux traitements thérapeutiques, et l'on peut citer à titre d'exemples la chirurgie obstétrique, digestive, viscérale, la chirurgie plastique et reconstructive, ainsi que le traitement des sciatalgies et des lombalgies.

## Revendications

1. Ceinture de contention dorso-abdominale, constituée par un ensemble unitaire à patte d'entre-jambes agencé pour exercer une compression permanente de la zone concernée du corps, caractérisée en ce que l'ensemble unitaire (11) se compose de deux parties complémentaires (12, 13) se raccordant par une couture associée (14), dont une partie principale antéro-postérieure enveloppante (12) réalisée en un premier matériau textile élastique choisi pour exercer une contention contrôlée, et une partie abdominale (13) réalisée en un second matériau textile élastique qui est sensiblement plus extensible que ledit premier matériau, de façon que cette partie abdominale (13) permette un développement plus libre de l'abdomen, et en ce qu'un bandeau périphérique (15) est relié à l'ensemble unitaire (11) par des coutures (16, 17) concernant la zone centrale antérieure et/ou la zone centrale postérieure de ce bandeau, le restant dudit bandeau reposant librement contre la surface dudit ensemble unitaire, afin que ce bandeau périphérique exerce une contention supplémentaire de la zone sus-pubienne et/ou pré-ombilicale.

2. Ceinture de contention selon la revendication 1, caractérisée en ce que la partie abdominale (13) de l'ensemble unitaire (11) se raccorde à la partie principale (12) dudit ensemble par une couture (14) en forme de U dont la partie incurvée est essentiellement recouverte par la partie frontale du bandeau périphérique (15).

3. Ceinture de contention selon la revendication 1 ou 2, caractérisée en ce que l'ensemble unitaire (11) comporte sur un côté une ligne d'ouverture à crochets (18) visant à faciliter la mise en place de ladite ceinture.

4. Ceinture de contention selon la revendication 3, caractérisée en ce que la ligne d'ouverture à crochets (18) comporte plusieurs rangées de façon à pouvoir régler le dimensionnement périmétrique de ladite ceinture.

5. Ceinture de contention selon l'une des revendications 1 à 4, caractérisée en ce que l'ensemble unitaire (11) est renforcé par un baleinage en vue d'un meilleur soutien, avec de préférence deux baleines antérieures (20) et deux baleines postérieures (21).

6. Ceinture de contention selon l'une des revendications 1 à 5, caractérisée en ce que les coutures (16, 17) reliant le bandeau périphérique (15) a l'ensemble unitaire (11) forment au moins une ligne de fixation centrale antérieure (16) et au moins une ligne de fixation centrale postérieure (17), ces deux lignes (16, 17) étant les seuls points de liaison entre ledit bandeau et ledit ensemble unitaire.

7. Ceinture de contention selon la revendication 6, caractérisée en ce que la ligne de fixation centrale antérieure (16) est agencée dans la zone sus-pubienne de l'ensemble unitaire (11), et la ligne de fixation centrale postérieure (17) est agencée dans la zone dorsale haute dudit ensemble unitaire.

8. Ceinture de contention selon l'une des revendications 1 à 7, caractérisée en ce que le bandeau périphérique (15) présente, de chaque côté, une ligne d'ouverture à crochets (22) à plusieurs rangées, de façon a pouvoir régler le dimensionnement périmétrique dudit bandeau.

9. Ceinture de contention selon l'une des revendications 1 à 8, caractérisée en ce que le bandeau périphérique (15) est réalisé en matériau textile élastique, ce matériau étant de préférence essentiellement identique au premier matériau constituant l'ensemble unitaire (11).

## Claims

1. A dorso-abdominal support belt constituted by unitary assembly having a crotch piece, and organized to exert permanent compression on the zone concerned of the body, characterized in that the unitary assembly (11) comprises two complementary portions (12, 13) connected together by an associated seam (14), said portions comprising an enveloping front-and-back main portion (12) made of a first elastic textile material selected to exert controlled support, and an abdominal portion (13) made of a second elastic textile material which is substantially more stretchable than said first material, such that said abdominal portion (13) allows freer swelling of the abdomen, and in that a peripheral band (15) is connected to the unitary assembly (11) via seams (16, 17) relating to the front middle zone and/or to the back middle zone of said band, the remainder of the band resting freely against the surface of said unitary assembly, in order that such peripheral band exerts additional support in the super-pubic and/or pre-umbilical zone.

2. A support belt according to claim 1, characterized in that the abdominal portion (13) of the unitary assembly (11) is connected to the main portion (12) of said assembly via a U-shaped seam (14) whose curved portion is essentially covered by the front portion of the peripheral band (15).

3. A support belt according to claim 1 or 2, characterized in that the unitary assembly (11) includes on one side a hook-fastened placket (18) for making said belt easier to put on.

4. A support belt according to claim 3, characterized in that the hook fastening (18) includes a plurality of rows of hooks so as to enable the circumferential size of said belt to be adjusted.

5. A support belt according to any one of claims 1 to 4, characterized in that the unitary assembly (11) is reinforced by stiffening in order to obtain better support, preferably with two front stiffeners (20) and two back stiffeners (21).

6. A support belt according to any one of claims 1 to 5, characterized in that the seams (16, 17) connecting the peripheral band (15) of the unitary assembly (11) form at least one front middle fixing line (16) and at least one back middle fixing line (17), said two fixing lines (16, 17) being the only points of connection between said band and said unitary assembly.

7. A support belt according to claim 6, characterized in that the front middle fixing line (16) is disposed in the super-pubic zone of the unitary assembly (11) and the back middle fixing line (17) is disposed in the top back zone of said unitary assembly.

8. A support belt according to any one of claims 1 to 7, characterized in that on each side the peripheral band (15) has a gap provided with a plurality of rows of hooks (22), thereby enabling the circumferential size of said band to be adjusted.

9. A support belt according to any one of claims 1 to 8, characterized in that the peripheral band (15) is made of an elastic textile material, said material being preferably essentially identical to the first material constituting the unitary assembly (11).

## Patentansprüche

1. Dorso abdominaler Stützgürtel, bestehend aus einer mit einem Schrittband versehenen Einheit zur Ausübung eines permanenten Druckes auf die betroffene Körperzone, dadurch **gekennzeichnet**, daß die Einheit (11) sich aus zwei komplementären Abschnitten, (12, 13) zusammensetzt, die durch eine zugehörige Naht (14) miteinander verbunden sind und von denen ein Vorder- und Rückseite umfassender Hauptabschnitt (12) aus einem ersten elastischen textilen Material besteht, das so ausgewählt ist, daß es eine kontrollierte Stützkraft ausübt, und von denen ein abdominaler Abschnitt (13) aus einem zweiten elastischen textilen Material besteht, das deutlich dehnbarer als das erste Material ist derart, daß dieser abdominale Abschnitt (13) eine freiere Ausdehnung des Bauches erlaubt, und daß ein umlaufender Gurt (15) mit der Einheit (11) über Nähte (16, 17) in der zentralen vorderen Zone und/oder der zentralen hinteren Zone dieses Gurtes verbunden ist, wobei der Gurt frei an der Fläche der Einheit anliegt, damit dieser umlaufende Gurt eine zusätzliche Stützung der über dem Schambein oder vor dem Nabel liegenden Zone bewirkt.

2. Stützgürtel nach Anspruch 1, dadurch **gekennzeichnet**, daß der abdominale Abschnitt (13) der Einheit mit dem Hauptabschnitt (12) dieser Einheit über eine U-förmige Naht (14) verbunden ist, deren gekrümmter Abschnitt im wesentlichen von dem Vorderabschnitt des umlaufenden Gurtes (15) bedeckt ist.

3. Stützgürtel nach Anspruch 1 oder 2, dadurch **gekennzeichnet**, daß die Einheit (11) an ihrer Seite eine mit Häkchen versehene Öffnungsleiste (18) hat, um das Anlegen des Gürtels zu erleichtern.

4. Stützgürtel nach Anspruch 3, dadurch **gekennzeichnet**, daß die mit Häkchen versehene Öffnungsleiste (18) mehrere Reihen umfaßt, um die Umfangsabmessung des Gürtels einstellen zu können.

5. Stützgürtel nach einem der Ansprüche 1 bis 4, dadurch **gekennzeichnet**, daß die Einheit (11) zur besseren Stützung durch eine Stabeinlage verstärkt ist mit vorzugsweise zwei vorderen Stäben (20) und zwei rückwärtigen Stäben (21).

6. Stützgürtel nach einem der Ansprüche 1 bis 5, dadurch **gekennzeichnet**, daß die den umlaufenden Gurt (15) mit der Einheit (11) verbindenden Nähte (16, 17) mindestens eine zentrale vordere Befestigungslinie (16) und mindestens eine zentrale rückwärtige Befestigungslinie (17) bilden, wobei die beiden Linien (16, 17) die einzigen Verbindungsstellen zwischen dem genannten Gurt und der Einheit sind.

7. Stützgürtel nach Anspruch 6, dadurch **gekennzeichnet**, daß die zentrale vordere Befestigungslinie (16) in der oberhalb des Schambeins liegenden Zone der Einheit (11) ausgebildet ist und das die zentrale rückwärtige Befestigungslinie (17) in der oberen Rückenpartie der Einheit ausgebildet ist.

8. Stützgürtel nach einem der Ansprüche 1 bis 7, dadurch **gekennzeichnet**, daß der umlaufende Gurt (15) auf jeder Seite eine mit mehreren Häkchenreihen versehene Öffnungsleiste (22) hat, um die Umfangsabmessung des Gurtes einstellen zu können.

9. Stützgürtel nach einem der Ansprüche 1 bis 8, dadurch **gekennzeichnet**, daß der umlaufende Gurt (15) aus einem elastischen textilen Material hergestellt ist, wobei dieses Material vorzugsweise mit dem zur Herstellung der Einheit (11) verwendeten ersten Material identisch ist.
